# EUROPEAN PATENT APPLICATION

(11) **EP 3 950 041 A1**
(43) Date of publication of application: **09.02.2022**
(21) Application number: 20784429.1
(22) Date of filing: 26.03.2020
(51) Int. Cl.: A61M 37/00

(54) **TRANSFER DEVICE FOR LIVING BODY**

(30) Priority: 29.03.2019 JP 2019068171
(71) Applicant: Toppan Printing Co., Ltd., Tokyo 110-0016 (JP)
(72) Inventor: IMAI Keiichi, Tokyo 110-0016 (JP); YOSHIHARA Kana, Tokyo 110-0016 (JP)
(74) Representative: TBK
(86) International application number: PCT/JP2020/013760
(87) International publication number: WO 2020/203675

(57) **Abstract**

A transfer device for living organisms is used for placing an object in a living organism. The transfer device for living organisms includes a tubular needle extending in one direction, and a pushing member located inside the needle. The needle is configured to capture an object therein from an opening at a tip of the needle. The pushing member is configured to be movable relative to the needle from inside the needle toward the opening.

## Description

### [Technical Field]

The present disclosure relates to transfer devices for living organisms used for transferring an object into living organisms.

### [Background Art]

Techniques have been developed for transplanting a transplant, which is obtained by culturing cells collected from a living organism, into an intradermal layer or subcutaneous layer of a living organism. For example, attempts are being made to regenerate hair by purifying cell groups that contribute to formation of hair follicles, which are organs producing hair, and transplanting the cell groups into an intradermal layer.

Hair follicles are formed by interactions between epithelial cells and mesenchymal cells. For good hair regeneration, it is desired that hair follicles having a normal structure and having periodic hair formation ability are generated from transplanted cell groups. Therefore, various research and development projects have been performed for methods of producing cell groups that can produce such hair follicles (e.g., see PTLs 1 to 3).

### [Citation List]

### [Patent Literature]

PTL1: WO2017/073625
PTL2: WO2012/108069
PTL3: JP 2008-29331 A

### [Summary of the Invention]

### [Technical Problem]

For smooth transplantation of a transplant, the transplant placed in a culture container or the like is desired to be smoothly transferred into a living organism. In tissue of living skins, organs, or the like, cells are so densely arranged that a large pressure is applied to a transplant from the tissue when placing the transplant in the tissue. Therefore, it has been desired to develop a device that assists placement of cells in living organisms.

This issue is not limited to transfer of cells but is common to the case in which a solid, such as a pharmaceutical, is transferred into a living organism and placed in tissue therein.

The present disclosure aims to provide a transfer device for living organisms capable of smooth placement of an object in a living organism.

### [Solution to Problem]

A transfer device for living organisms for solving the above issue is a transfer device for living organisms that places an object in a living organism, including: a tubular needle extending in one direction, the needle being configured to capture the object into the needle from an opening at a tip of the needle; and a pushing member located inside the needle and configured to be movable relative to the needle from inside the needle toward the opening.

With the above configuration, movement of the pushing member relative to the needle can push the object accommodated in the needle out of the opening of the needle while the object is held by the pushing member. Thus, compared to the case where an object is pushed out of the needle using only liquid flow, the object can be easily pushed out of the needle into tissue of a living organism. This can achieve smooth placement of an object at a target site.

### [Advantageous Effects of the Invention]

According to the present disclosure, an object can be smoothly placed in a living organism.

### [Brief Description of the Drawings]

Fig. 1 is a diagram illustrating a general configuration of a cell transplantation device, according to a first embodiment of a cell transplantation device as a transfer device for living organisms.
Fig. 2 is a diagram illustrating a cross-sectional structure of a needle and a pushing member according to the cell transplantation device of the first embodiment.
Fig. 3 is a diagram illustrating an example of a general configuration of the cell transplantation device of the first embodiment.
Fig. 4 is a diagram illustrating a cross-sectional structure of a cell transplantation device including a plurality of needles, according to the cell transplantation device of the first embodiment.
Fig. 5 is a diagram illustrating an arrangement of a cell transplantation device and a tray holding a transplant, in a transplantation method using the cell transplantation device of the first embodiment.
Fig. 6 is a diagram illustrating a process of holding a transplant, in the transplantation method using the cell transplantation device of the first embodiment.
Fig. 7 is a diagram illustrating a process of accommodating a transplant, in the transplantation method using the cell transplantation device of the first embodiment.
Fig. 8 is a diagram illustrating a process of piercing a needle into a transplantation site, in the transplantation method using the cell transplantation device of the first embodiment.
Fig. 9 is a diagram illustrating a process of placing a transplant, in the transplantation method using the cell transplantation device of the first embodiment.
Fig. 10 is a diagram illustrating another example of an arrangement of a cell transplantation device and a tray holding a transplant, in the transplantation method using the cell transplantation device of the first embodiment.
Fig. 11 is a diagram illustrating another example of the process of holding a transplant, in the transplantation method using the cell transplantation device of the first embodiment.
Fig. 12 is a diagram illustrating another example of the process of accommodating a transplant, in the transplantation method using the cell transplantation device of the first embodiment.
Fig. 13 is a diagram illustrating a cross-sectional structure of a needle and a pushing member according to a second embodiment of a cell transplantation device as a transfer device for living organisms.
Fig. 14 is a diagram illustrating a process of accommodating a transplant, in a transplantation method using a cell transplantation device of the second embodiment.
Fig. 15 is a diagram illustrating a process of piercing a needle into a transplantation site, in the transplantation method using the cell transplantation device of the second embodiment.
Fig. 16 is a diagram illustrating a process of placing a transplant, in the transplantation method using the cell transplantation device of the second embodiment.
Fig. 17 is a diagram illustrating a cross-sectional structure of a needle and a pushing member according to a third embodiment of a cell transplantation device as a transfer device for living organisms.
Fig. 18 is a diagram illustrating a process of accommodating a transplant, in a transplantation method using a cell transplantation device of the third embodiment.
Fig. 19 is a diagram illustrating a process of piercing a needle into a transplantation site, in the transplantation method using the cell transplantation device of the third embodiment.
Fig. 20 is a diagram illustrating a process of placing a transplant, in the transplantation method using the cell transplantation device of the third embodiment.

### [Description of the Embodiments]

### (First Embodiment)

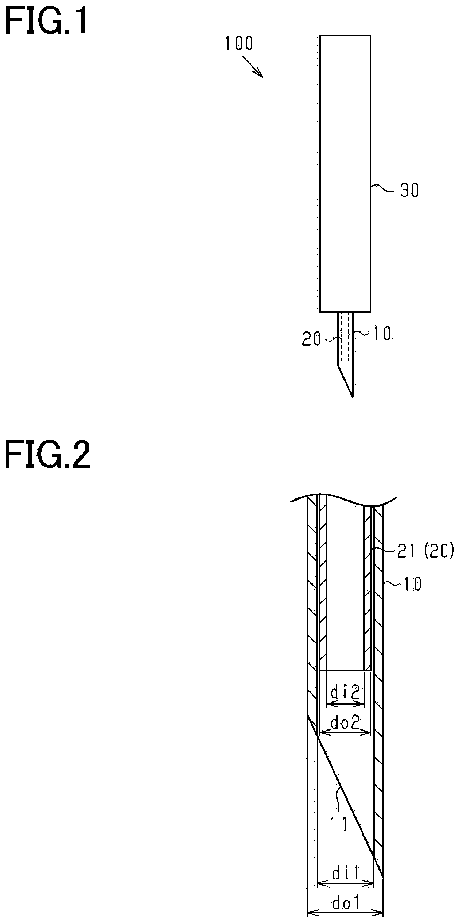

Referring to Figs. 1 to 12, a first embodiment of a transfer device for living organisms will be described. The transfer device for living organisms of the present embodiment is embodied in a cell transplantation device used for cell transplantation.

### [Object to be transplanted]

The cell transplantation device of the present embodiment is used for transplanting a transplant into a living organism. The transplantation target site is on the inside of at least an intradermal layer or a subcutaneous layer, or on the inside of organ tissue or the like. The transplant includes cell groups. Such a cell group as an object to be transplanted will be described.

A cell group to be transplanted includes a plurality of cells. The cell group may be an aggregate of a plurality of aggregated cells, or may be an aggregate of a plurality of cells that are connected by intercellular junctions. Alternatively, the cell group may be formed of a plurality of dispersed cells. Further, cells included in the cell group may be undifferentiated cells, or may be cells that have been differentiated. Alternatively, the cell group may include both undifferentiated cells and differentiated cells. The cell group may include, for example, masses of cells (spheroids), primordia, tissue, organs, organoids, and mini organs.

The cell group as an object to be transplanted has an ability to act on tissue formation in a living organism when placed at a target site. An example of such a cell group is a cellular aggregate containing cells having the nature of stem cells. The cell group may contribute, for example, to hair growth or hair restoration by being placed in an intradermal layer or subcutaneous layer. Specifically, the cell group may have an ability to function as a hair follicle organ, an ability to differentiate into a hair follicle organ, an ability to induce or promote formation of a hair follicle organ, or an ability to induce or promote formation of hair in hair follicles. Furthermore, the cell group may include cells, such as pigment cells or stem cells that differentiate into pigment cells, contributing to controlling hair color. Also, the cell group may include vascular cells.

Specifically, an example of the cell group as an object to be transplanted in the present embodiment is a hair follicle primordium, which is a primitive hair follicle organ. The hair follicle primordium includes mesenchymal cells and epithelial cells. In a hair follicle organ, hair dermal papilla cells, which are mesenchymal cells, induce differentiation of hair follicle epithelial stem cells to form hair bulbs, so that hair matrix cells in the hair bulbs repeat division to form hair. The hair follicle primordium is a cell group that differentiates into such a hair follicle organ.

The hair follicle primordium may be formed, for example, by culturing a mixture of mesenchymal cells and epithelial cells under predetermined conditions. The mesenchymal cells are derived from mesenchymal tissue such as hair dermal papilla, and the epithelial cells are derived from epithelial tissue located in a bulge region or hair bulb bases. It should be noted that the method of forming a hair follicle primordium is not limited to the above example. Also, the mesenchymal cells and epithelial cells used for forming a hair follicle primordium may also be derived from any piece of tissue. That is, these cells may be derived from a hair follicle organ, or may be derived from an organ different from the hair follicle organ, or may be derived from pluripotent stem cells.

It should also be noted that a transplant may contain not only a cell group but also components assisting transplantation of the cell group.

### [Cell transplantation device]

Fig. 1 shows a cell transplantation device 100 including a needle 10 extending in one direction, a pushing member 20 located inside the needle 10, and a position changer 30 having a structure for moving the needle 10 and the pushing member 20 relatively to each other.

A detailed configuration of the needle 10 and the pushing member 20 will be described first. As shown in Fig.2, the needle 10 has a tubular shape. The pushing member 20 includes an inner tube 21 located inside the needle 10. The needle 10 may have any shape as long as it has a tip that is capable of piercing a transplantation target site of a living organism. For example, the needle 10 may extend in a cylindrical shape and may have a sharp tip as a result of being cut obliquely with respect to the direction in which the needle 10 extends.

The inner tube 21 located inside the needle 10 extends in the direction in which the needle 10 extends. As long as the inner tube 21 has a tubular shape, it may have any other shape elements. For example, if the needle 10 extends in a cylindrical shape, the inner tube 21 may also extend in a cylindrical shape. The tip of the inner tube 21 is preferred to have an end face in a plane perpendicular to the direction in which the inner tube 21 extends. The needle 10 and the inner tube 21 are formed of metal or resins.

The inner tube 21 is configured to be movable relative to the needle 10 in the direction in which the inner tube 21 and the needle 10 extend. Specifically, it is configured so that the position of the inner tube 21 is changeable with respect to the needle 10 between a position defining a first state in which the tip of the inner tube 21 is located inside the needle 10 without protruding from an opening 11 at the tip of the needle 10 and a position defining a second state in which the tip of the inner tube 21 protrudes from the opening 11 and located outside the needle 10.

Furthermore, a suction force is ensured to be caused inside the inner tube 21 with the operation of a mechanism connected to the inner tube 21. It should be noted that the position changer 30 may have a function of causing a suction force inside the inner tube 21. Instead of causing a suction force inside the inner tube 21, a pneumatic pressure may be applied to the inside of the inner tube 21.

The inner tube 21 attracts a transplant using the suction force caused inside the inner tube 21 to hold the transplant at the tip of the inner tube 21. The inner tube 21 has an inner diameter not allowing the whole transplant to enter the inner tube 21. However, the inner diameter of the inner tube 21 is preferred to have a length allowing a sufficient suction force to be exerted to attract a transplant. There may be a gap between the inner peripheral surface of the needle 10 and the outer peripheral surface of the inner tube 21 so that the inner tube 21 can move parallel to the needle 10.

For example, if the transplant is a cell group contributing to hair growth or hair restoration, i.e., an aggregate of cells such as a cellular aggregate, the diameter of the cell group when approximated to a sphere, i.e., the diameter of the minimum sphere circumscribing the cell group, may be approximately 0.1 mm or more and 1 mm or less.

In this case, an inner diameter di2 of the inner tube 21 may be selected, for example, from the range of 0.05 mm or more and 1.1 mm or less, and an inner diameter di1 of the needle 10 may be selected, for example, from the range of 0.2 mm or more and 1.5 mm or less.

As an example, if the approximate diameter of the cell group as a transplant is in the range of around 0.1 mm to 0.2 mm, the inner diameter di2 of the inner tube 21 may be 0.1 mm, an outer diameter do2 of the inner tube 21 may be 0.2 mm, the inner diameter di1 of the needle 10 may be 0.22 mm, and an outer diameter do1 of the needle 10 may be 0.4 mm. In this case, the needle 10 may be a 27G injection needle.

As another example, if the approximate diameter of the cell group as a transplant is in the range of around 0.2 mm to 0.3 mm, the inner diameter di2 of the inner tube 21 may be 0.17 mm, the outer diameter do2 of the inner tube 21 may be 0.36 mm, the inner diameter di1 of the needle 10 may be 0.4 mm, and the outer diameter do1 of the needle 10 may be 0.5 mm. In this case, the needle 10 may be a 25G injection needle.

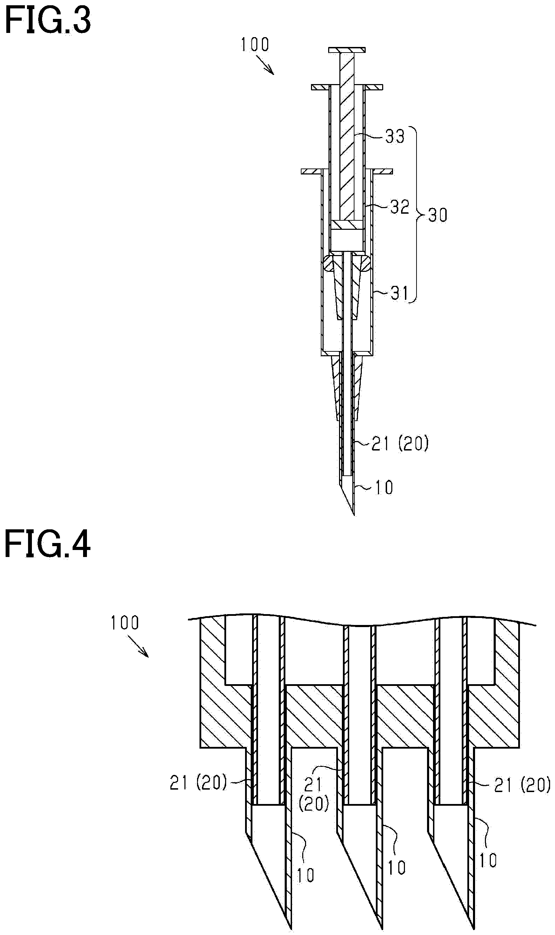

Fig. 3 shows an example of a cell transplantation device 100 including a position changer 30 enabling movement of the inner tube 21 with respect to the needle 10. The cell transplantation device 100 has a structure in which two syringes are stacked. A first syringe barrel 31, that is an outer syringe barrel, has a tip to which the base of the needle 10 is connected, and a second syringe barrel 32, that is a syringe barrel inserted into the first syringe barrel 31, has a tip to which the base of the inner tube 21 is connected. The inner tube 21 extends from inside the first syringe barrel 31 to the inside of the needle 10.

The second syringe barrel 32 serves as a plunger that pushes the first syringe barrel 31. When the second syringe barrel 32 is moved inside the first syringe barrel 31 in the direction in which the needle 10 extends, the position of the inner tube 21 with respect to the needle 10 is changed.

On the inside of the second syringe barrel 32, a plunger 33 is inserted to serve as a member that pushes the second syringe barrel 32. When the plunger 33 is pulled back inside the second syringe barrel 32 in the direction in which the needle 10 extends, a suction force is caused inside the inner tube 21, and when the plunger 33 is pushed, pressure is applied to the inside of the inner tube 21.

In the above configuration, the first syringe barrel 31, the second syringe barrel 32, and the plunger 33 configure the position changer 30. The position changer 30 also has a function of causing a suction force inside the inner tube 21 and applying pressure inside the inner tube 21.

The position changer 30 may have any configuration as long as the inner tube 21 is movable with respect to the needle 10. The inner tube 21 may be manually moved with respect to the needle 10 or may be electrically moved using a motor or the like. Suction force or pressure may be produced inside the inner tube 21 by driving a machine, such as a compressor, connected to the inner tube 21.

The number of the needles 10 provided to the cell transplantation device 100 is not particularly limited, but may be one as in the cell transplantation device 100 shown in Figs. 1 and 3 or may be plural as shown in Fig. 4. If the cell transplantation device 100 includes a plurality of needles 10, the arrangement of these needles 10 is not particularly limited, but they may be arranged regularly or irregularly.

If the cell transplantation device 100 includes a plurality of needles 10, the inner tubes 21 may be moved independently with respect to the respective needles 10 or the inner tubes 21 may be moved simultaneously with respect to the needles 10. Furthermore, a suction force may be caused independently inside the plurality of inner tubes 21 or may be caused simultaneously.

### [Transplantation method]

A transplantation method for a transplant using the cell transplantation device 100 of the first embodiment will be described.

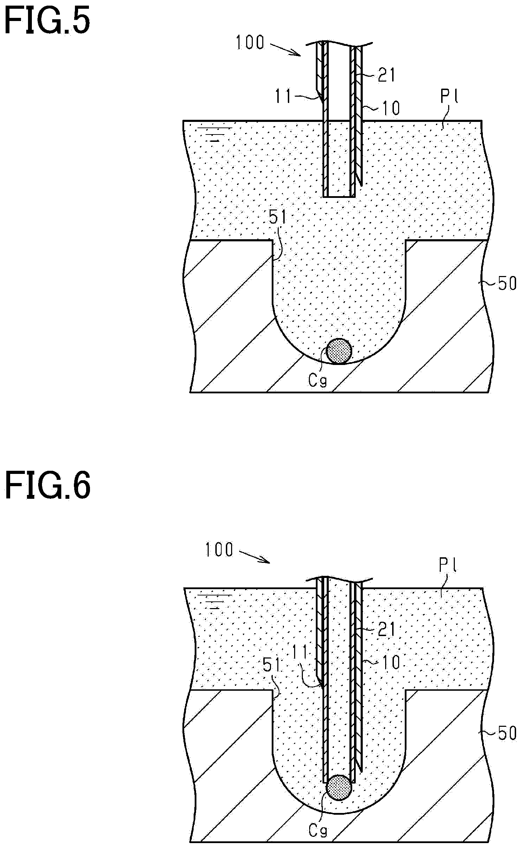

As shown in Fig. 5, a transplant Cg before being transplanted is held in a tray 50, such as a culture container, together with a protective solution Pl. For example, as shown in Fig. 5, the transplant Cg is placed in a recess 51 of the tray 50. The protective solution Pl is filled in the recess 51 and in the entire region above the recess 51 in the tray 50. The method of holding the transplant Cg and the protective solution Pl in the tray 50 is not limited to the method in which the protective solution Pl is filled in and held by the entire tray 50 including the recess 51 and the transplant Cg is held in the recess 51. For example, the transplant Cg and the protective solution Pl may be held on a flat surface of the tray 50.

The protective solution Pl may be any solution that is unlikely to hinder the viability of cells, and is preferred to be a solution that has only a small influence on a living organism when injected into the living organism. For example, the protective solution Pl may be a saline solution, a solution that protects skin, such as Vaseline or lotion, or a mixture of these solutions. The protective solution Pl may contain additional components, such as nutritional components. If the tray 50 is a culture container and the transplant Cg is cultured in the tray 50, the protective solution Pl may be a culture medium for culturing cells or may be a liquid exchanged from the culture medium. The liquid that contains the transplant Cg and the protective solution Pl may be a low viscosity fluid or a high viscosity fluid.

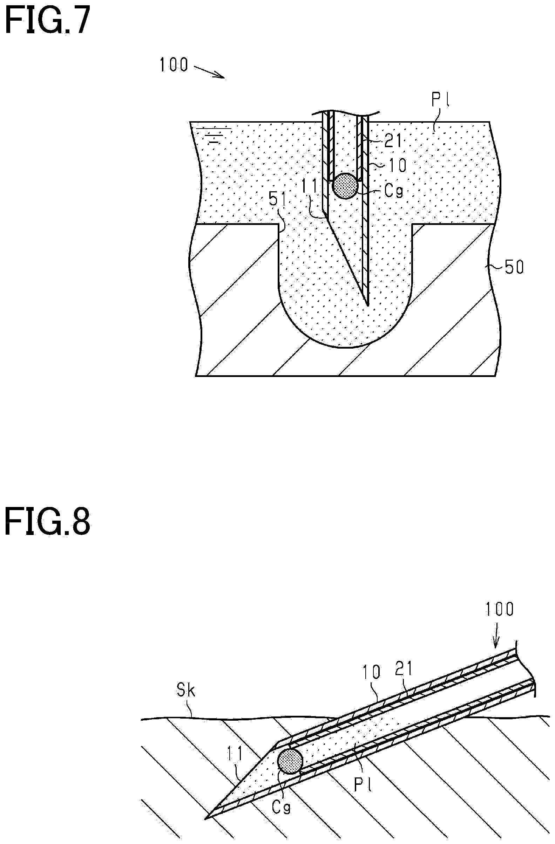

When capturing a transplant Cg into the needle 10, the cell transplantation device 100 is in a state in which the tip of the inner tube 21 protrudes from the opening 11 at the tip of the needle 10. In this state, the tip of the inner tube 21 is brought close to the transplant Cg in the protective solution Pl and a suction force is caused inside the inner tube 21. Thus, as shown in Fig. 6, the transplant Cg is attracted to the tip of the inner tube 21 to hold the transplant Cg at the tip of the inner tube 21. After that, as shown in Fig. 7, the inner tube 21 is moved relative to the needle 10 so that the tip of the inner tube 21 is pulled into the needle 10. With this movement, the transplant Cg is accommodated inside the needle 10. It is preferred that the transplant Cg is captured together with the protective solution Pl so that the protective solution Pl is present around the transplant Cg inside the needle 10.

The cell transplantation device 100 accommodating the transplant Cg in the needle 10 is brought to a transplantation site, e.g., a skin Sk, of a living organism, and the needle 10 is pierced, as shown in Fig. 8, into the transplantation site. In this case, since the transplant Cg and the tip of the inner tube 21 are accommodated inside the needle 10, the tip of the needle 10 is located at the end of the cell transplantation device 100. Since the tip of the needle 10 has a shape facilitating piercing into a living organism, the cell transplantation device 100 smoothly enters the transplantation site.

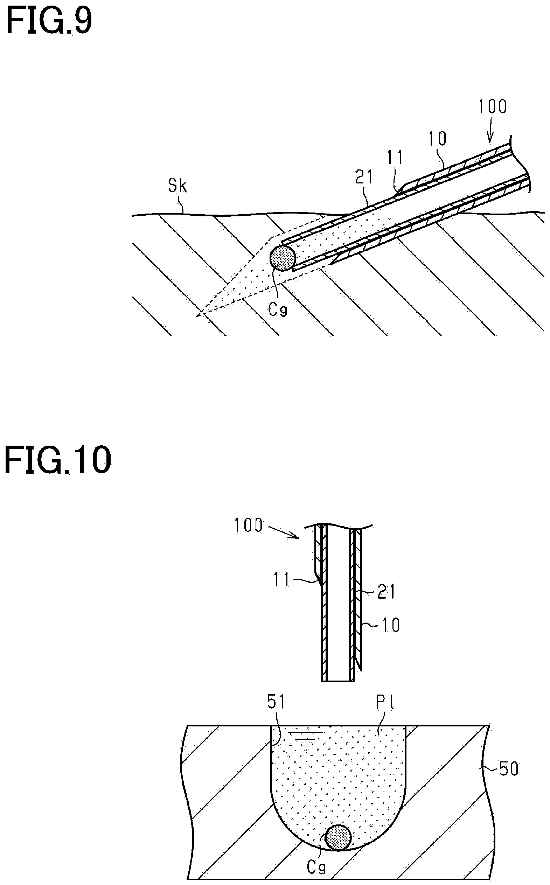

When the tip of the needle 10 enters the transplantation target site, the needle 10 is pulled back, as shown in Fig. 9, relative to the inner tube 21. Specifically, the needle 10 is retracted in the direction in which it is pulled out of the transplantation site, but the positions of the inner tube 21 and the transplant Cg held at the tip of the inner tube 21 remain unchanged. Thus, the transplant Cg and the tip of the inner tube 21 protrude from the opening 11 of the needle 10 and, consequently, the transplant Cg and the tip of the inner tube 21 are positioned in the space of the transplantation site where the tip of the needle 10 was located. Retention of the transplant Cg is released due to the transplant Cg contacting or being affected by the surrounding tissue and thus the transplant Cg is separated from the tip of the inner tube 21 and placed at the transplantation target site. To assist release of retention of the transplant Cg, pressure may be applied inside the inner tube 21. After that, the needle 10 and the inner tube 21 are pulled out of the transplantation site.

Thus, transplantation of the transplant Cg into the target site is completed. According to the transplantation method using the cell transplantation device 100, a single device can perform the series of processes starting from capturing a transplant Cg from a tray 50 until placing the transplant Cg at a transplantation target site. Therefore, cells can be transplanted smoothly.

Since the inner tube 21 can be moved relative to the needle 10 so as to protrude from the opening 11 of the needle 10, the transplant Cg is pushed out of the needle 10 while being held at the tip of the inner tube 21 and is placed at the target site. For example, if the transplant Cg is attempted to be placed at a target site only using liquid flow such as of the protective solution PI, the transplant Cg is less likely to flow out of the needle 10 because the liquid penetration rate is slow due to the cells being densely arranged in tissue of a living organism. In this regard, in the present embodiment, the transplant Cg is easily pushed out of the needle 10 due to the movement of the needle 10 and the inner tube 21 relative to each other, while being held at the tip of the inner tube 21 as a structural object. Accordingly, the transplant Cg can be smoothly placed at a target site.

In particular, the needle 10 is pulled back relative to the inner tube 21 in a direction from the tip of the needle 10 toward the base thereof, so that the tip of the inner tube 21 protrudes from the opening 11 of the needle 10. Therefore, the transplant Cg can be placed in the space where the tip of the needle 10 was located. Specifically, since the space for placing the transplant Cg is formed in advance by the tip of the needle 10, the pressure applied to the transplant Cg by the tissue is reduced when placing the transplant Cg. Therefore, the magnitude of the pressure applied to the transplant Cg by the tissue can be reduced.

Also, compared to the placement of a transplant Cg at a target site using only liquid flow, the placement position of the transplant Cg can be easily controlled in the direction parallel to the surface of the transplantation site and in the depth direction of the transplantation site. Specifically, the transplant Cg can be placed at a location following the position of the tip of the inner tube 21. Accordingly, the placement position of the transplant Cg can be reliably controlled by controlling the position of the tip of the inner tube 21. Specifically, when placing the transplant Cg at a target site by pulling back the needle 10 relative to the inner tube 21, the placement position of the transplant Cg can be controlled by controlling the location of the needle 10 and the tip of the inner tube 21, i.e., by controlling the position and depth of piercing the needle 10 and controlling the position and depth of the tip of the inner tube 21.

Furthermore, when capturing a transplant Cg into the needle 10, a suction force is caused inside the inner tube 21 to hold the transplant Cg at the tip of the inner tube 21. Accordingly, the transplant Cg can be suitably continuously held at the tip of the inner tube 21 while the transplant Cg is accommodated inside the needle 10 and when the transplant Cg is pushed out of the needle 10. Therefore, placement of the transplant Cg at the target site after being pushed out of the needle 10 can be reliably achieved by the relative movement of the inner tube 21 to the needle 10.

Furthermore, when capturing the transplant Cg into the needle 10, the transplant Cg is held at the tip of the inner tube 21 in a state in which the tip of the inner tube 21 protrudes from the opening 11 of the needle 10. Accordingly, compared to the case where the transplant Cg is held at the tip of the inner tube 21 on the inside of the needle 10, the tip of the inner tube 21 can be brought closer to the transplant Cg located in the tray 50 when holding the transplant Cg. This can achieve smooth holding of the transplant Cg.

Figs. 8 and 9 show an example in which the needle 10 penetrates into the transplantation site in a direction oblique to the surface of the transplantation site; however, the needle 10 may penetrate into the transplantation site in a direction perpendicular to the surface of the transplantation site.

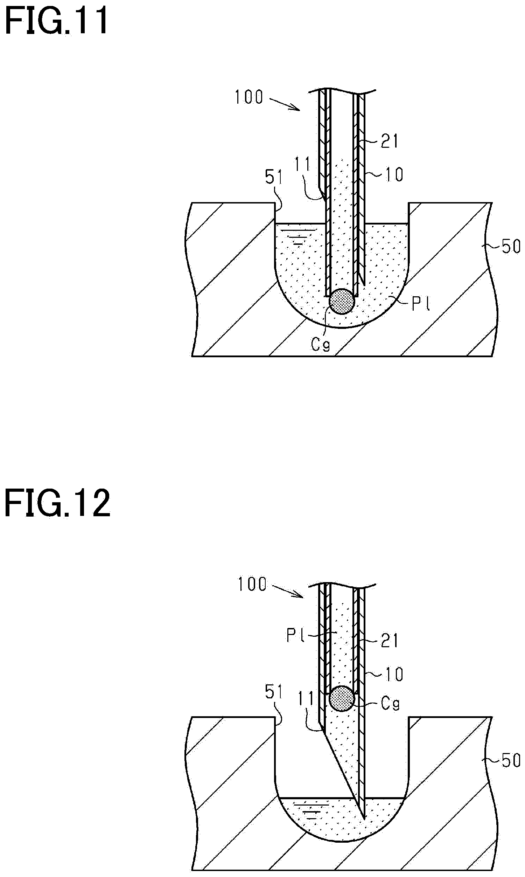

Furthermore, as shown in Fig. 10, the protective solution Pl may be held only in the recess 51 of the tray 50. In this case as well, a suction force is caused inside the inner tube 21 that is brought close to the transplant Cg in the protective solution Pl so that, as shown in Fig. 11, the transplant Cg can be held at the tip of the inner tube 21. After that, as shown in Fig. 12, the inner tube 21 is moved into the needle 10 to accommodate the transplant Cg inside the needle 10.

The transplant Cg may be captured in a state in which the tip of the inner tube 21 is positioned inside the needle 10. Specifically, a suction force may be caused inside the inner tube 21 in a state in which the tip of the inner tube 21 is positioned inside the needle 10, so that the transplant Cg is sucked into the needle 10 and held at the tip of the inner tube 21. In this case, a suction mechanism may be connected to the needle 10 so that a suction force can be caused inside the needle 10 instead of inside the inner tube 21. Thus, a suction force may be caused inside the needle 10 to cause a suction force in the gap between the needle 10 and the inner tube 21, so that the transplant Cg can be sucked into the needle 10. Furthermore, the protective solution Pl may be present or may not be present inside the inner tube 21 while the transplant Cg is accommodated inside the needle 10. Whether the protective solution Pl enters the inner tube 21 depends on, for example, in which of the needle 10 and the inner tube 21 a suction force is caused.

As described above, the transfer device for living organisms of the first embodiment can achieve the advantageous effects as follows.
(1) Relative movement of the pushing member 20 toward the opening 11 of the needle 10 can push the transplant Cg accommodated in the needle 10 out of the opening 11 of the needle 10 while the transplant Cg is held by the pushing member 20. Thus, compared to the case where the transplant Cg is pushed out of the needle 10 using only liquid flow, the transplant Cg can be easily pushed out of the needle 10 into tissue. This can achieve smooth placement of a transplant Cg at a target site.
(2) The needle 10 is pulled back relative to the pushing member 20 in a direction from the tip of the needle 10 toward the base thereof, so that the tip of the pushing member 20 protrudes further than the needle 10. Therefore, the transplant Cg can be placed in the space where the tip of the needle 10 was located. Accordingly, the pressure applied to the transplant Cg from tissue can be reduced when placing the transplant Cg.
(3) The transplant Cg can be placed at a location following the position of the tip of the pushing member 20. Accordingly, compared to the case where the transplant Cg is placed using only liquid flow, the placement position of the transplant Cg can be easily controlled through control of the position of the tip of the pushing member 20.
(4) The pushing member 20 includes the inner tube 21 and is configured to be movable relative to the needle 10 between a position defining a first state in which the tip of the inner tube 21 is located inside the needle 10 and a position defining a second state in which the tip of the inner tube 21 protrudes from the opening 11 of the needle 10. With this configuration, the pushing member 20 is moved relative to the needle 10 until the tip of the inner tube 21 protrudes from the opening 11 of the needle 10. Therefore, the transplant Cg is held by the pushing member 20 until it is completely outside the needle 10. This can achieve smoother placement of a transplant Cg at a target site.
(5) The inner tube 21 is configured to be connected to a mechanism causing a suction force inside the inner tube 21 and be capable of holding the transplant Cg at the tip of the inner tube 21 by the suction force. With this configuration, since the transplant Cg is held at the tip of the inner tube 21, retention of the transplant Cg can be reliably maintained by the pushing member 20 from when the transplant Cg is accommodated in the needle 10 until when it is placed at a target site. Therefore, the transplant Cg can be reliably placed at a target site by the movement of the pushing member 20 relative to the needle 10.
(6) When capturing the transplant Cg into the needle 10, the transplant Cg is held at the tip of the inner tube 21 in a state in which the tip of the inner tube 21 protrudes from the opening 11 of the needle 10. Accordingly, compared to the case where the transplant Cg is held at the tip of the inner tube 21 on the inside of the needle 10, the tip of the inner tube 21 can be brought closer to the transplant Cg located in the tray 50 when holding the transplant Cg. This can achieve smooth holding of the transplant Cg.
(7) A cell transplantation device 100 including a pushing member 20 that is movable relative to a needle 10 can be reliably achieved with a simple configuration if the device includes a first syringe barrel 31 having a tip to which the needle 10 is connected and a second syringe barrel 32 having a tip to which the pushing member 20 is connected, with the second syringe barrel 32 inserted into the first syringe barrel 31.
(8) When the transplant Cg is a cellular aggregate and the needle 10 is configured to capture a liquid containing the cellular aggregate into needle 10, the cellular aggregate can be smoothly placed at a target site when transplanted therein.

### (Second Embodiment)

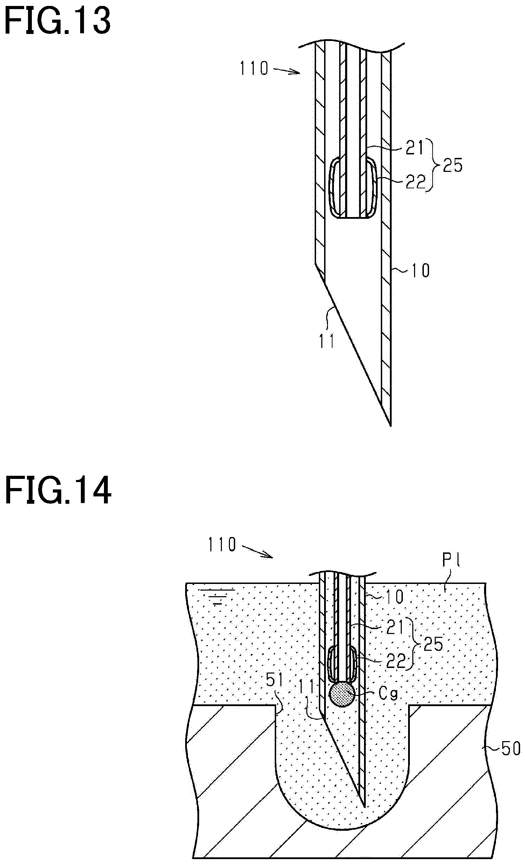

Referring to Figs. 13 to 16, a second embodiment of the transfer device for living organisms will be described. The transfer device for living organisms of the second embodiment is also embodied in a cell transplantation device used for transplantation of a transplant, as in the first embodiment. The second embodiment is different from the first embodiment in the configuration of the pushing member. The following description is focused on differences of the second embodiment from the first embodiment, and therefore like components of the first embodiment are designated with like reference signs to omit repeated explanation.

### [Cell transplantation device]

As shown in Fig. 13, the second embodiment is provided with a pushing member 25 which includes not only a tubular inner tube 21 located inside a needle 10 but also an inflation part 22 mounted to a tip of the inner tube 21. The inflation part 22 inflates with injection of air. Specifically, the inflation part 22 has a bag- or balloon-shaped structure and is deflated before a transplant is accommodated. The inflation part 22 is formed of, for example, a biocompatible material.

The inner tube 21 is configured to be movable together with the inflation part 22 relative to the needle 10 in the direction in which the needle 10 extends. Specifically, it is configured so that the position of the pushing member 25 is changeable with respect to the needle 10 between a position defining a first state in which the tip of the inner tube 21 and the inflation part 22 are located inside the needle 10 without protruding from an opening 11 at the tip of the needle 10 and a position defining a second state in which the tip of the inner tube 21 and the inflation part 22 protrude from the opening 11 of the needle 10 and located outside the needle 10.

When a mechanism connected to the inner tube 21 is operated, a suction force can be caused inside the inner tube 21 and at the same time air can be injected into the inflation part 22 through the inner tube 21. It should be noted that the position changer 30 may have a function of causing a suction force inside the inner tube 21 and injecting air into the inflation part 22.

The first embodiment described above is provided with the position changer 30 which is configured by the first syringe barrel 31, the second syringe barrel 32, and the plunger 33. This mode may be applied to the second embodiment.

### [Transplantation method]

A transplantation method for a transplant using a cell transplantation device 110 of the second embodiment will be described. As in the first embodiment, a transplant Cg can be held at the tip of the inner tube 21 in a state in which the tip of the inner tube 21 protrudes from the opening 11 at the tip of the needle 10 and a suction force is caused inside the inner tube 21. Then, as shown in Fig. 14, the tip of the inner tube 21 is pulled into the needle 10. With this movement, the transplant Cg is accommodated inside the needle 10.

As in the first embodiment, a suction force may be caused inside the inner tube 21 in a state in which the tip of the inner tube 21 is located inside the needle 10, so that the transplant Cg is sucked into the needle 10 and held at the tip of the inner tube 21. Also, a suction force may be caused inside the needle 10 to cause a suction force in the gap between the needle 10 and the inner tube 21, so that the transplant Cg can be sucked into the needle 10. Furthermore, the protective solution Pl may be present or may not be present inside the inner tube 21 while the transplant Cg is accommodated inside the needle 10. Whether the protective solution Pl enters the inner tube 21 depends on, for example, in which of the needle 10 and the inner tube 21 a suction force is caused, or how the inflation part 22 is mounted to the tip of the inner tube 21.

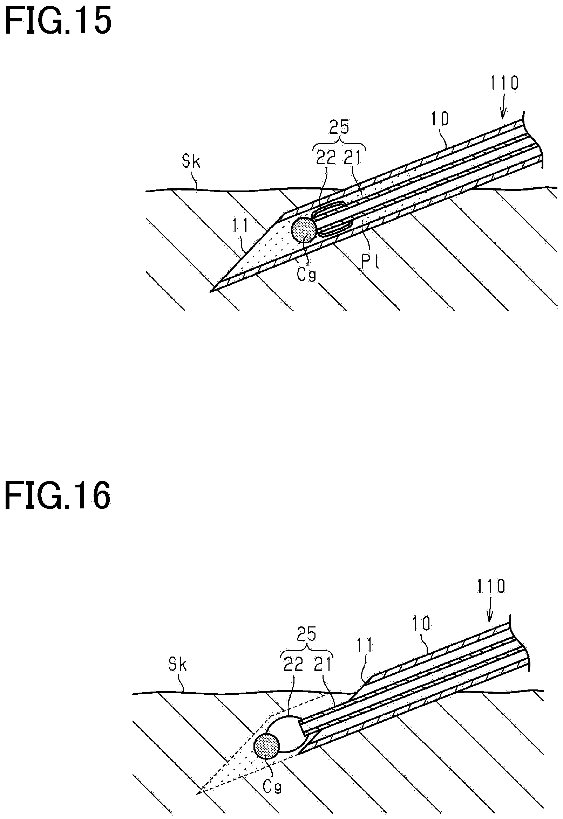

The cell transplantation device 110 accommodating the transplant Cg in the needle 10 is brought to a transplantation site, e.g., a skin Sk, of a living organism and the needle 10 is pierced, as shown in Fig. 15, into the transplantation site. When the tip of the needle 10 enters the transplantation target site, the needle 10 is pulled back, as shown in Fig. 16, relative to the inner tube 21. Then, the inflation part 22 is inflated by injecting air into the inner tube 21. Thus, the transplant Cg, the tip of the inner tube 21, and the inflation part 22 protrude from the opening 11 of the needle 10. Consequently, retention of the transplant Cg by the inner tube 21 is released, so that the transplant Cg that has been held by the inflation part 22 is placed at the transplantation target site. After that, the needle 10 and the inner tube 21 are pulled out of the transplantation site. The inflation part 22 may be retracted from the transplantation site together with the needle 10 and the inner tube 21, or may be left inside the transplantation site.

Thus, transplantation of the transplant Cg into the target site is completed. In the second embodiment as well, since the pushing member 25 formed of the inner tube 21 and the inflation part 22 can be moved relative to the needle 10 so as to protrude from the opening 11 of the needle 10, the transplant Cg is pushed out of the needle 10 while being held at the tip of the pushing member 25 and is placed at the target site. This can achieve smooth placement of a transplant Cg at a target site.

In the second embodiment, the inflation part 22 pushes tissue to form a space therein. Therefore, a space for placing a transplant Cg can be suitably secured to enable smoother placement of the transplant Cg at the target site.

In the second embodiment, at least the inflated inflation part 22 may protrude from the opening 11 of the needle 10 when placing the transplant Cg at a target site. In other words, the tip of the inner tube 21 may be located inside the needle 10. As long as the inflated inflation part 22 protrudes from the opening 11 of the needle 10, the transplant Cg that has been held at the tip of the pushing member 25 can be placed at the target site.

As described above, according to the transfer device for living organisms of the second embodiment, the following advantageous effects can be achieved in addition to (1) to (8) of the first embodiment.

(9) The pushing member 25 includes the inner tube 21 and the inflation part 22. With this configuration, the inflated inflation part 22 pushes tissue to form a space therein, and therefore a space for placing the transplant Cg can be suitably secured. This can achieve smoother placement of a transplant Cg at a target site.

### (Third Embodiment)

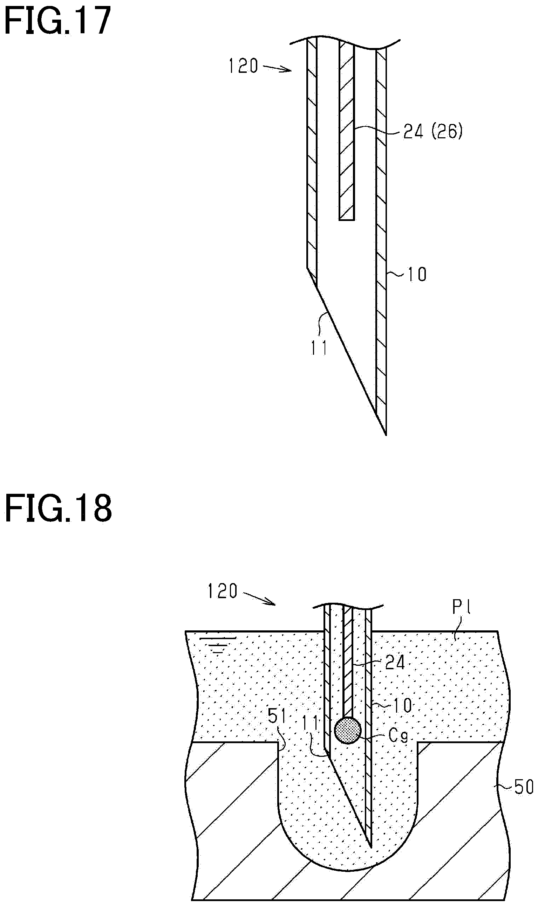

Referring to Figs. 17 to 20, a third embodiment of the transfer device for living organisms will be described. The transfer device for living organisms of the third embodiment is also embodied in a cell transplantation device used for transplantation of a transplant, as in the first embodiment. The third embodiment is different from the first embodiment in the configuration of the pushing member. The following description is focused on differences of the third embodiment from the first embodiment, and therefore like components of the first embodiment are designated with like reference signs to omit repeated explanation.

### [Cell transplantation device]

As shown in Fig. 17, a pushing member 26 of the third embodiment includes a rod 24 located inside a needle 10. The rod 24 located inside the needle 10 extends in the direction in which the needle 10 extends. The rod 24 may have any shape as long as it has a structure extending like a rod with no space inside. There is a gap formed between the rod 24 and the needle 10 with a size disabling entry of a transplant Cg.

The rod 24 is configured to be movable relative to the needle 10 in the direction in which the needle 10 extends. Specifically, it is configured so that the position of the rod 24 with respect to the needle 10 is changeable between a position defining a first state in which a tip of the rod 24 is located inside the needle 10 without protruding from an opening 11 at the tip of the needle 10 and a position defining a second state in which the tip of the rod 24 protrudes from the opening 11 of the needle 10 and located outside the needle 10.

Furthermore, when a mechanism connected to the needle 10 is operated, a suction force is ensured to be caused inside the needle 10. It should be noted that the position changer 30 may have a function of causing a suction force inside the needle 10. Instead of causing a suction force inside the needle 10, a pneumatic pressure may be applied to the inside of the needle 10.

The first embodiment described above is provided with the position changer 30 which is configured by the first syringe barrel 31, the second syringe barrel 32, and the plunger 33. This mode may be applied to the third embodiment. These components may be assembled such that a suction force can be caused inside the needle 10 through the gap between the needle 10 and the rod 24 by pulling back the second syringe barrel 32 or the plunger 33.

### [Transplantation method]

A transplantation method for a transplant using a cell transplantation device 120 of the third embodiment will be described.

In the third embodiment, the tip of the rod 24 is located inside the needle 10 when capturing a transplant Cg into the needle 10. Then, as shown in Fig. 18, when the tip of the needle 10 is oriented to the transplant Cg and a suction force is caused inside the needle 10, the transplant Cg is accommodated in the needle 10 from the opening 11 together with the protective solution PI. Since the rod 24 is located inside the needle 10, the transplant Cg is retained in a region between the tip of the rod 24 and the opening 11 inside the needle 10.

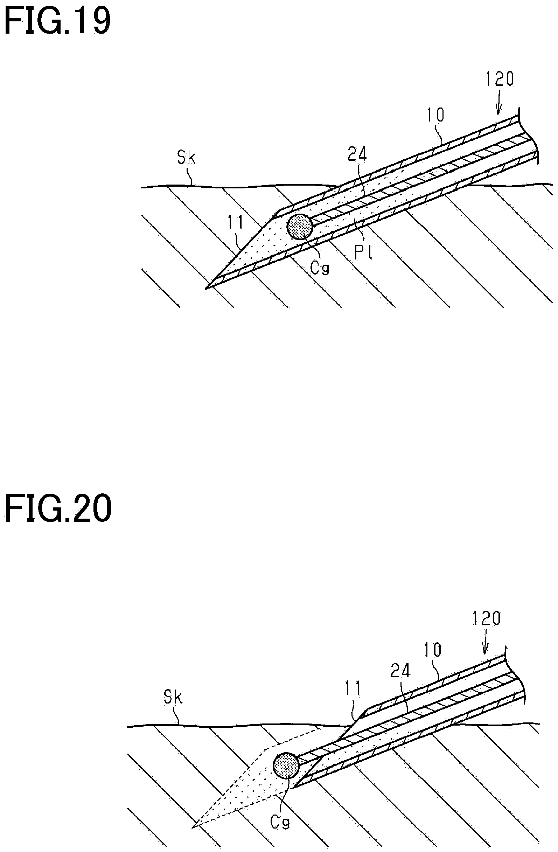

The cell transplantation device 120 accommodating the transplant Cg in the needle 10 is brought to a transplantation site, e.g., a skin Sk, of a living organism and the needle 10 is pierced, as shown in Fig. 19, into the transplantation site. When the tip of the needle 10 enters the transplantation target site, the needle 10 is pulled back, as shown in Fig. 20, relative to the rod 24. Consequently, the transplant Cg and the tip of the rod 24 protrude from the opening 11 at the tip of the needle 10, so that the transplant Cg and the tip of the rod 24 are located in the space of the transplantation site where the tip of the needle 10 was located. In this way, the transplant Cg is placed at the transplantation target site. After that, the needle 10 and the rod 24 are pulled out of the transplantation site.

Thus, transplantation of the transplant Cg into the target site is completed. In the third embodiment as well, since the pushing member 26 formed of the rod 24 can be moved relative to the needle 10 so as to protrude from the opening 11 of the needle 10, the transplant Cg is pushed out of the needle 10 while being held at the tip of the rod 24 and is placed at the target site. This can achieve smooth placement of a transplant Cg at a target site.

As described above, according to the transfer device for living organisms of the third embodiment, the following advantageous effects can be achieved in addition to (1) to (3), (7) and (8) of the first embodiment.

(10) It is configured so that the pushing member 26 includes the rod 24 and is movable relative to the needle 10 between a position defining a first state in which the tip of the rod 24 is located inside the needle 10 and a position defining a second state in which the tip of the rod 24 protrudes from the opening 11 of the needle 10. With this configuration, the pushing member 26 is moved relative to the needle 10 until the tip of the rod 24 protrudes from the opening 11 of the needle 10. Therefore, the transplant Cg is held by the pushing member 26 until it is completely outside the needle 10. This can achieve smoother placement of a transplant Cg at a target site.

### (Examples)

The above cell transplantation device as a transfer device for living organisms will be described using a specific example. The cell transplantation device of the example corresponds to the cell transplantation device of the first embodiment.

### [Formation of cell transplantation device]

A cell transplantation device as shown in Fig. 3 was formed, with a structure in which two syringe barrels were stacked. A 25G injection needle was used as a needle. The needle had an inner diameter of 0.40 mm and an outer diameter of 0.50 mm. A precision tube having an inner diameter of 0.17 mm and an outer diameter of 0.36 mm was used as an inner tube.

### [Evaluation]

Using the cell transplantation device of the example, the performance of placing a transplant at a target site was evaluated by conducting two kinds of tests.

### <Test 1>

Plastic beads having an average particle size of 210 µm were prepared as models of transplants. In a test, one plastic bead was sucked and held by the inner tube of the cell transplantation device and transferred to the inside of a skin isolated from a nude mouse. This test was conducted ten times.

After the tests, the front and the rear of each isolated skin were observed to confirm whether the plastic bead had been placed inside the skin. Consequently, it was confirmed that in all of the ten tests, the plastic bead was fixed inside the isolated skin without being left on the inside of the needle. Thus, it was confirmed that a transplant could be placed inside a skin using the cell transplantation device of the example.

### <Test 2>

Hair follicle primordia fixed by formalin were prepared as transplants. In a test, as in Test 1, one hair follicle primordium was sucked and held by the inner tube of the cell transplantation device and transferred to the inside of a skin isolated from a nude mouse. This test was conducted ten times. A cell group (4×10³ epithelial cells and 4×10³ mesenchymal cells) having an average diameter of less than around 400 µm was used as a hair follicle primordium.

After the tests, the front and the rear of each isolated skin were observed to confirm whether the hair follicle primordium had been placed inside the skin. Consequently, it was confirmed that in all of the ten tests, the hair follicle primordium was fixed inside the isolated skin without being left on the inside of the needle. Thus, it was confirmed that a transplant could be placed inside a skin using the cell transplantation device of the example.

### (Modifications)

The embodiments described above can be modified and implemented as follows.

When placing a transplant at a target site, the needle 10 does not always have to be pulled back in a direction from the tip of the needle 10 toward the base thereof. Instead, the pushing member may be moved relative to the needle 10 from inside the needle 10 toward the opening 11. For example, after entry of the needle 10 into a transplantation site, only the pushing member may be moved to a position where it protrudes from the opening 11 without changing the position of the needle 10. In this case, before moving the pushing member, the needle 10 is preferred to be slightly retracted together with the pushing member in the direction in which the needle 10 is pulled out of the transplantation site. Thus, a transplant can be placed in the space where the tip of the needle 10 was located. Also, for example, after entry of the needle 10 into a transplantation site, the needle 10 and the pushing member may be moved in opposite directions so that the pushing member protrudes from the opening 11.

In the embodiments described above, the tubular member, i.e., the needle 10 or the inner tube 21, is not limited to have a structure extending in a cylindrical shape as long as the tubular member has openings at the tip and the base and these openings communicate with each other through the space inside the tubular member. The tubular member may have any outer shape such as a columnar, conical, or prismatic shape. The space defined inside the tubular member may have any shape such as a columnar, conical, or prismatic shape.

The cell group to be transplanted does not necessarily have to be a cell group that contributes to hair growth or hair restoration, but may be any cell group that exhibits desired effects when placed in tissue of a living organism. For example, the cell group to be transplanted may be a cell group that exhibits effects in cosmetic use, such as elimination of wrinkles or improvement of moisturizing state in the skin. Furthermore, an object transferred into a living organism by the transfer device for living organisms is not limited to a cell group, but may be a solid object such as a pharmaceutical.

## Claims

1. A transfer device for living organisms that places an object in a living organism, comprising:
a tubular needle extending in one direction, the needle being configured to capture the object into the needle from an opening at a tip of the needle; and
a pushing member located inside the needle and configured to be movable relative to the needle from inside the needle toward the opening.

2. The transfer device for living organisms according to claim 1, wherein
the pushing member includes a tubular inner tube extending in a direction in which the needle extends; and
the pushing member is configured to be movable relative to the needle between a position defining a first state in which a tip of the inner tube is located inside the needle and a position defining a second state in which the tip of the inner tube protrudes from the opening of the needle.

3. The transfer device for living organisms according to claim 2, wherein
the inner tube is connected to a mechanism causing a suction force inside the inner tube; and
the inner tube is configured so as to be capable of holding the object at the tip of the inner tube using the suction force.

4. The transfer device for living organisms according to any one of claims 1 to 3, wherein the pushing member comprises:
a tubular inner tube extending in a direction in which the needle extends; and
an inflation part located at a tip of the inner tube and inflated by injection of air.

5. The transfer device for living organisms according to claim 1, wherein
the pushing member includes a rod extending in a direction in which the needle extends; and
the pushing member is configured to be movable relative to the needle between a position defining a first state in which a tip of the rod is located inside the needle and a position defining a second state in which the tip of the rod protrudes from the opening of the needle.

6. The transfer device for living organisms according to any one of claims 1 to 5, wherein
the device comprises a first syringe barrel and a second syringe barrel;
the first syringe barrel has a tip to which the needle is connected;
the second syringe barrel has a tip to which the pushing member is connected; and
the second syringe barrel is inserted into the first syringe barrel.

7. The transfer device for living organisms according to any one of claims 1 to 6, wherein
the object is a cellular aggregate; and
the needle is configured to capture a liquid containing the cellular aggregate into the needle.
